# EUROPEAN PATENT SPECIFICATION

(11) **EP 1 467 778 B1**
(45) Date of publication and mention of the grant of the patent: **12.08.2009**
(21) Application number: 02784406.7
(22) Date of filing: 06.11.2002
(51) Int. Cl.: A61M 25/00, A61M 25/10

(54) **GUIDEWIRE OCCLUSION SYSTEM UTILIZING REPEATABLY INFLATABLE GAS-FILLED OCCLUSIVE DEVICE**
FÜHRUNGSDRAHT-OKKLUSIONSSYSTEM MIT WIEDERHOLT AUFFÜLLBARER GASGEFÜLLTER OKKLUSIONSVORRICHTUNG
SYSTEME D'OCCLUSION POUR FIL-GUIDE, UTILISANT UN DISPOSITIF D'OCCLUSION REMPLI DE GAZ POUVANT ETRE GONFLE ET DEGONFLE DE MANIERE SELECTIVE

(30) Priority: 06.11.2001 US 12903; 06.11.2001 US 12891; 06.11.2001 US 7788
(43) Date of publication of application: 20.10.2004
(73) Proprietor: Medrad, Inc., Indianola, PA 15051 (US)
(72) Inventor: BONNETTE, Michael, J., Minneapolis, MN 55433-8003 (US); THOR, Eric, J., Minneapolis, MN 55433-8003 (US); JENSEN, Mark, L., Greenfield, MN 55357-9571 (US); KRAVIK, Richard, C., Champli, MN 55316 (US); LE, Hieu, V., Minneapolis, MN 55433-8003 (US)
(74) Representative: Browne, Robin Forsythe
(86) International application number: PCT/US2002/035633
(87) International publication number: WO 2003/039624

(56) References cited:
- WO-A-01/62329
- WO-A-99/42161
- US-A- 4 758 223
- US-A- 6 036 715
- US-A- 6 123 698
- US-A1- 2001 014 821
- US-B1- 6 190 354
- US-B1- 6 251 093

## Description

### FIELD OF THE INVENTION

The present invention relates generally to the field of vascular medical devices. More specifically, the present invention relates to a guidewire occlusion system for use in vascular procedures that uses a repeatably inflatable gas-filled occlusive device.

### BACKGROUND OF THE INVENTION

Arterial disease involves damage that happens to the arteries in the body. Diseased arteries can become plugged with thrombus, plaque, or grumous material that may ultimately lead to a condition known as ischemia. Ischemia refers to a substantial reduction or loss of blood flow to the heart muscle or any other tissue that is being supplied by the artery and can lead to permanent damage of the affected region. While arterial disease is most commonly associated with the formation of hard plaque and coronary artery disease in the heart, similar damage can happen to many other vessels in the body, such as the peripheral vessels, cerebral vessels, due to the buildup of hard plaque or softer thrombus or grumous material within the lumen of an artery or vein.

A variety of vascular medical devices and procedures have been developed to treat diseased vessels. The current standard procedures include bypass surgery (where a new blood vessel is grafted around a narrowed or blocked artery) and several different types of non-surgical interventional vascular medical procedures, including angioplasty (where a balloon on a catheter is inflated inside a narrowed or blocked portion of an artery in an attempt to push back plaque or thrombotic material), stenting (where a metal mesh tube is expanded against a narrowed or blocked portion of an artery to hold back plaque or thrombotic material), and debulking techniques in the form of atherectomy (where some type of high speed or high power mechanism is used to dislodge hardened plaque) or thrombectomy (where some type of mechanism or infused fluid is used to dislodge grumous or thrombotic material). In each of these interventional vascular medical procedures, a very flexible guidewire is routed through the patient's vascular system to a desired treatment location and then a catheter that includes a device on the distal end appropriate for the given procedure is tracked along the guidewire to the treatment location.

Although interventional vascular procedures avoid many of the complications involved in surgery, there is a possibility of complications if some of the plaque, thrombus or other material breaks free and flows downstream in the artery or other vessel, potentially causing a stroke, a myocardial infarction (heart attack), or other tissue death. One solution to this potential complication is to use some kind of occlusive device to block or screen the blood flowing downstream of the treatment location. Examples of catheter arrangements that use a pair of balloons as occlusive devices to create an isolated space in the blood vessel are described in U.S. Patent Nos. 4,573,966, 4,636,195, 5,059,178, 5,320,604, 5,833,644, 5,925,016, 6,022,336 and 6,176,844. Examples of catheter arrangements that use a single balloon as an occlusive device either upstream or downstream of the treatment location are described in U.S. Patent Nos. 5,171,221, 5,195,955, 5,135,482, 5,380,284, 5,688,234, 5,713,917, 5,775,327, 5,792,179, 5,807,330, 5,833,650, 5,843,022, 6,021,340, 6,159,195 and 6,248,121. An example of a catheter arrangement that uses a mechanically-expanded occlusive device is shown in U.S. Patent No. 6,231,588. Occlusive balloons also have been used on non-over-the-wire catheters without any guidewire internal to the catheter as described, for example, in U.S. Patent Nos. 4,838,268 and 5,209,727.

The use of an occlusive device as part of a vascular procedure is becoming more common in debulking procedures performed on heart bypass vessels. Most heart bypass vessels are harvested and transplanted from the saphenous vein located along the inside of the patient's leg. The saphenous vein is a long, straight vein that has a capacity more than adequate to support the blood flow needs of the heart. Once transplanted, the saphenous vein is subject to a buildup of plaque or thrombotic materials in the grafted arterial lumen. Unfortunately, the standard interventional vascular treatments for debulking are only moderately successful when employed to treat saphenous vein coronary bypass grafts. The complication rate for a standard balloon angioplasty procedure in a saphenous vein coronary bypass graft is higher than in a native vessel with the complications including embolization, "no-reflow" phenomena, and procedural related myocardial infarction. Atherectomy methods including directional, rotational, and laser devices are also associated with a high degree of embolization resulting in a greater likelihood of infarction. The use of stents for saphenous vein coronary bypass grafts has produced mixed results. Stents provide for less restenosis, but they do not eliminate the risk of embolization and infarction incurred by standard balloon angioplasty.

In order to overcome the shortcomings of these standard non-surgical interventional treatments in treating saphenous vein coronary bypass graft occlusion, embolic protection methods utilizing a protective device distal to the lesion have been developed. The protective device is typically a filter or a balloon. Use of a protective device in conjunction with an atherectomy or thrombectomy device is intended to prevent emboli from migrating beyond the protective device and to allow the embolic particles to be removed, thereby subsequently reducing the risk of myocardial infarction. When the occlusive device is a balloon, the balloon is inserted and inflated at a point distal to the treatment site or lesion site. Therapy is then performed at the treatment site and the balloon acts to block all blood flow which prevents emboli from traveling beyond the balloon. Following treatment, some form of particle removal device must be used to remove the dislodged emboli prior to balloon deflation. U.S. Patent No. 5,843,022 uses a balloon to occlude the vessel distal to a lesion or blockage site. The occlusion is treated with a high pressure water jet, and the fluid and entrained emboli are subsequently removed via an extraction tube. U.S. Patent 6,135,991 describes the use of a balloon to occlude the vessel allowing blood flow and pressure to prevent the migration of emboli proximally from the treatment device.

There are various designs that have included an occlusive balloon on the end of a guidewire. U.S. Patent Nos. 5,520,645, 5,779,688 and 5,908,405 describe guidewires having removable occlusive balloons on a distal end. U.S. Patent No. 4,573,470 describes a guidewire having an occlusive balloon where the guidewire is bonded inside the catheter as an integral unit. U.S. Patent Nos. 5,059,176, 5,167,239, 5,520,645, 5,779,688 and 6,050,972 describe various guidewires with balloons at the distal end in which a valve arrangement is used to inflate and/or deflate the balloon. U.S. Patent No. 5,908,405 describes an arrangement with a removable balloon member that can be repeatedly inserted into and withdrawn from a guidewire. U.S. Patent No. 5,776,100 describes a guidewire with an occlusive balloon adhesively bonded to the distal end with an adapter on the proximal end to provide inflation fluid for the occlusive balloon.

Except in the case of the normal cerebral anatomy where there are redundant arteries supplying blood to the same tissue, one of the problems with using an occlusive device in the arteries is that tissue downstream of the occlusive device can be damaged due to the lack of blood flow. Consequently, an occlusive device that completely blocks the artery can only be deployed for a relatively short period of time. To overcome this disadvantage, most of the recent development in relation to occlusive devices has focused on devices that screen the blood through a filter arrangement. U.S. Patent Nos. 5,827,324, 5,938,672, 5,997,558, 6,080,170, 6,171,328, 6,203,561 and 6,245,089 describe various examples of filter arrangements that are to be deployed on the distal end of a catheter system. While a filter arrangement is theoretically a better solution than an occlusive device, in practice such filter arrangements often become plugged, effectively turning the filter into an occlusive device. The filter arrangements also are mechanically and operationally more complicated than an occlusive balloon device in terms of deployment and extraction.

As is the case in almost all angioplasty devices or stenting catheter devices where a balloon is used to expand the blood vessel or stent, most catheter occlusive balloons as well as most guidewire occlusive balloons utilize a liquid fluid such as saline or saline mixed with a radiopaque marker for fluoroscopic visualization (i.e., contrast) as the inflation medium. Generally, a liquid fluid medium for expanding vascular balloons has been preferred because the expansion characteristics of a liquid are more uniform and predictable, and because a liquid medium is easier to work with and more familiar to the doctors. In the case of angioplasty balloons, for example, high-pressure requirements (up to 20 atmospheres) necessitate that the inflation fluid be an incompressible fluid for safety reasons. While having numerous advantages, liquid fluids do not lend themselves to rapid deflation of an occlusive balloon because of the high resistance to movement of the liquid in a long small diameter tube. In the context of angioplasty procedures, the balloon catheter has a much larger lumen than a guidewire. Consequently, rapid deflation is possible. In the context of a guidewire, however, liquid filled occlusive balloons typically cannot be deflated in less than a minute and, depending upon the length of the guidewire, can take up to several minutes to deflate. Consequently, it is not practical to shorten the period of total blockage of a vessel by repeatedly deflating and then re-inflating a liquid filled occlusive balloon at the end of a guidewire.

Gas-filled balloons have been used for intra-aortic occlusive devices where rapid inflation and deflation of the occlusive device is required. Examples of such intra-aortic occlusive devices are shown in U.S. Patent Nos. 4,646,719, 4,733,652, 5,865,721, 6,146,372, 6,245,008 and 6,241,706. While effective for use as an intra-aortic occlusive device, these occlusive devices are not designed for use as a guidewire as there is no ability to track a catheter over the intra-aortic occlusive device.

An early catheter balloon device that utilized a gas as an inflation medium and provided a volume limited syringe injection system is described in U.S. Patent No. 4,865,587. More recently, a gas-filled occlusive balloon on a guidewire is described as one of the alternate embodiments in U.S. Patent No. 6,217,567. The only suggestion for how the guidewire of the alternate embodiment is sealed is a valve type arrangement similar to the valve arrangement used in a liquid fluid embodiment. A similar gas-filled occlusive balloon has been described with respect to the Aegis Vortex ^{TM} system developed by Kensey Nash Corporation In both U.S. Patent No. 6,217,567 and the Aegis Vortex™ system, the gas-filled occlusive balloon is used for distal protection to minimize the risk of embolization while treating a blocked saphenous vein coronary bypass graft. Once deployed, the occlusive balloon retains emboli dislodged by the atherectomy treatment process until such time as the emboli can be aspirated from the vessel. No specific apparatus are shown or described for how the gas is to be introduced into the device or how the occlusive balloon is deflated.

Although the use of occlusive devices has become more common for distal embolization protection in vascular procedures, particularly for treating a blocked saphenous vein coronary bypass graft, all of the existing approaches have significant drawbacks that can limit their effectiveness. Liquid filled occlusive balloons can remain in place too long and take too long to deflate, increasing the risk of damages downstream of the occlusion. Occlusive filters are designed to address this problem, but suffer from blockage problems and can be complicated to deploy and retrieve and may allow small embolic particles to migrate downstream. Existing gas-filled occlusive balloons solve some of the problems of liquid filled occlusive balloons, but typically have utilized complicated valve and connection arrangements. It would be desirable to provide for an occlusive device that was effective, sample, quick to deploy and deflate, and that could overcome the limitations of the existing approaches.

WO01/62329 discloses a guidewire occlusive system having an occlusive balloon. a gas inflation evacuation system, a gas inflation/evacuation system and a sealing system including means for selectively sealing an extended sealable section to form an airtight seal.

WO99/42161 discloses a plug system for a low profile hollow catheter comprising a tubular guidewire assembly having an occlusive balloon proximate a distal end and an extended sealable portion proximate a proximal end, a guidewire inflation/evacuation system being removably connectable to the proximal end of the assembly.

### SUMMARY OF THE INVENTION

The present invention is a guidewire occlusion system for use in vascular procedures that includes a repeatably inflatable gas-filled occlusive balloon or other occlusive device proximate a distal end of a tubular guidewire assembly having an extended sealable section at a proximal end. A gas inflation/evacuation system is removably connectable to the proximal end of the tubular guidewire assembly and includes an evacuation syringe to evacuate the tubular guidewire assembly and an inflation syringe or syringes for introducing a gas under pressure into the tubular guidewire assembly to inflate the occlusive balloon or other occlusive device a plurality of times. A sealing system is also removably connectable to the proximal end of the tubular guidewire assembly and selectively seals the tubular guidewire assembly at one of a plurality of separate locations along the extended sealable section to form an airtight seal of the tubular guidewire assembly. Each time a deflation of the occlusive balloon is desired in order to reestablish blood flow to the vessel downstream of the occlusive balloon, the proximal end of the extended sealable section preferably is cut distal to the location of the last seal to quickly deflate the occlusive balloon.

The advantage of the guidewire occlusion system of the present invention is that the occlusive device can be repeatably inflated and deflated a plurality of times during a vascular procedure in between which the proximal end of the tubular guidewire assembly is free of mechanical connections and obstructions and, therefore, the tubular guidewire assembly can function as a conventional exchange guidewire assembly for one or more over-the-wire catheters. Alternatively, the tubular guidewire assembly of the present invention can be shorter in length for use with rapid exchange catheter systems. Unlike operation of existing liquid filled occlusive devices, the present invention enables repeated and quick inflation and deflation which allows an operator to deploy the gas-filled occlusive device numerous times during a procedure for shorter periods of time, thereby reducing the risk of potential damage to downstream tissue. Unlike operation of other gas-filled occlusive devices, the simplicity of the present invention permits the tubular guidewire assembly to be used as a conventional exchange guidewire assembly. There are no complicated mechanical arrangements or valve systems internal to the tubular guidewire assembly that increase the cost, complexity, and potential for failure of the system.

The extended sealable section is an extended crimpable section and the sealing system includes a crimping mechanism. The extended crimpable section has a sufficient length to permit a plurality of crimps and cuts along the extended crimpable section and preferably has an outer diameter that is smaller than the outer diameter of the main body portion of the guidewire assembly. The crimping mechanism is used to crimp the extended crimpable section of the guidewire assembly to seal the guidewire assembly a plurality of times. Preferably, the gas inflation/evacuation system and the crimping mechanism and sealing mechanism of the sealing system constitute a handheld apparatus. Each time a deflation of the occlusive device is desired in order to reestablish blood flow to the vessel downstream of the occlusive device, the extended crimpable section is cut distal to the location of the last crimp so as to quickly deflate the occlusive device. Preferably, the extended crimpable section of the guidewire assembly is dimensioned and the crimping mechanism is arranged such that an effective outer diameter of the extended crimpable section at the location of a seal is no greater than the outer diameter of the main body portion of the guidewire assembly when the extended crimpable section is sealed.

In one embodiment for coronary vascular procedures, the guidewire assembly preferably has an effective length of at least 40 cm and more preferably at least 100 cm and an outer diameter of less than 1.5 mm (0.060 inch) and more preferably less than 0.5 mm (0.018 inch), the extended sealable section has an effective length of at least 1 cm and more preferably at least 5 cm and an outer diameter of less than 1_3 mm (0.050 inch) and more preferably less than 3 m (0.012 inch), and the occlusive device (balloon) is deflated in less than two minutes and more preferably less than one minute. This embodiment is particularly adapted to provide distal embolization protection in debulking vascular interventional procedures, such as those involving a blocked saphenous vein coronary bypass graft.
Alternatively, the guidewire assembly may be configured and dimensioned for use in peripheral vascular procedures or neurovascular procedures.

In a preferred embodiment, the inflation system of the gas inflation/evacuation system includes a plurality of individually actuatable syringes each containing a sufficient volume of biocompatible gas for a single inflation of the occlusive device so as to minimize the volume of biocompatible gas in the gas inflation/evacuation system in the event of a leak. The preferred embodiment is packaged in a sterile packaging that is assembled and packaged in a sealed chamber filled with a biocompatible gas such that any gas within the sterile packaging once packaged is only the biocompatible gas.

### BRIEF DESCRTPTION OF THE DRAWINGS

Fig. 1 is a schematic diagram of a guidewire occlusion system in accordance with the present invention and operating in an evacuation mode.
Fig. 2 is a schematic diagram of the guidewire occlusion system shown in Fig. 1 operating in an inflation mode.
Fig. 3a is a side view of the guidewire assembly shown in Fig. 1; Fig. 3b is an enlarged view of the portion of Fig. 3a delineated by the circle 3b; Fig. 3c is an enlarged cross-sectional view of the occlusive balloon at the distal end of the guidewire assembly; and Fig. 3d is a detailed view of the distal portion of the guidewire assembly absent the occlusive balloon.
Figs. 4a and 4b are fragmentary cross-sectional views of different manners of joining the extended sealable section to the main body portion at the proximal portion of the guidewire assembly of Fig. 3a.
Figs. 5-7 are perspective views of three alternate embodiments of gas inflation/evacuation systems and the sealing systems used therewith.
Fig. 8 is an exploded view of the gas inflation/evacuation system of the alternate embodiment shown in Fig. 7 and the associated sealing system.
Fig. 9 is a perspective view of the sealing system illustrated with the alternate embodiment shown in Fig.7.
Fig. 10 is a top view of a preferred embodiment of a gas inflation/evacuation system and sealing system used in the present invention.
Fig. 11 is a perspective view of another alternate embodiment of a gas inflation/evacuation system and sealing system.
Fig. 12 is an end view of a crimping mechanism.
Figs. 13 and 14 are two sectional views of the crimping mechanism of Fig. 12, Fig. 14 being a view taken along the line 14-14 of Fig. 12, and Fig. 13 being a magnification of the portion of Fig. 14 indicated by the dashed circle.
Fig. 15 is a cross-sectional view of a sealing system showing a plugging mechanism that does not for part of the invention.
Fig. 16 is a schematic view of equipment including a sealed chamber for use in assembling and packaging the guidewire occlusion system.
Fig. 17 is a side view of a biocompatible packaging.
Fig. 18 is an exploded view of still another alternate embodiment of a gas inflation/evacuation system and sealing system.
Fig. 19 is a partially exploded view of the alternate embodiment of Fig. 18 including the entire joinable housing assembly thereof.
Figs. 20 and 21 are side views of alternate embodiments of the guidewire assembly shown in Fig. 3a.

### DETAILED DESCRIPTION OF THE PREFERRED EMBODIMENTS

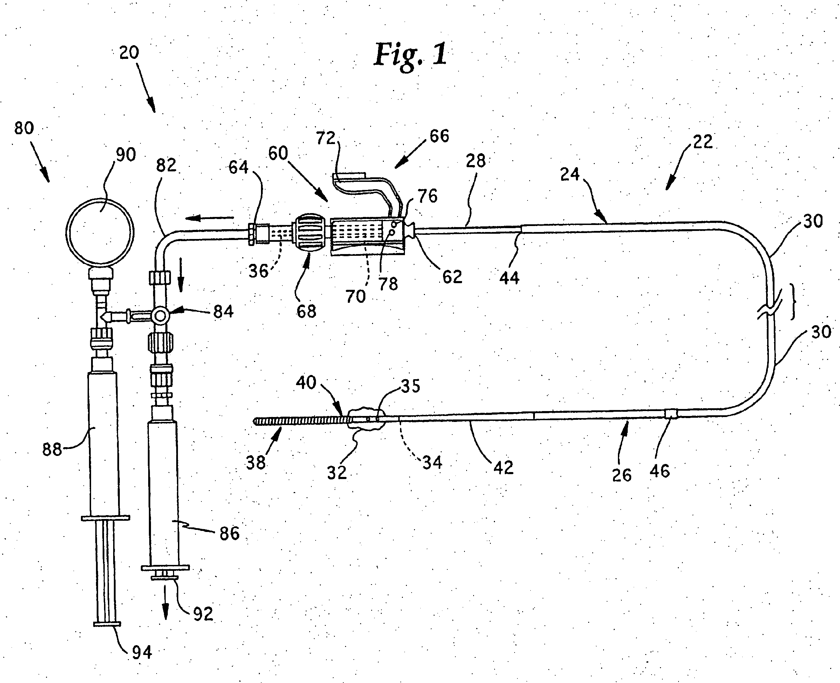

Referring now to Figs. 1-2, the overall structure and operation of a guidewire occlusion system 20 in accordance with the present invention will be described. The guidewire occlusion system 20 includes a guidewire assembly 22, a sealing system 60, and a gas inflation/evacuation system 80.

Guidewire assembly 22 is a tubular member that includes a proximal portion 24 and a distal portion 26.
As used in the present invention, the terms proximal and distal will be used with reference to an operator, such that a distal portion of the guidewire assembly 22, for example, is the portion first inserted into a blood vessel, and the proximal portion remains exterior to the patient and is therefore closer to the operator. An extended sealable section 28 is provided proximate the proximal portion 24 of guidewire assembly 22.
The extended sealable section 28 is an extended crimpable section comprised of a tubular segment having an outer diameter smaller than an outer diameter of a main body portion 30 of guidewire assembly 22. Although the diameter of the extended crimpable section could be any size consistent with effective use as a guidewire, it will be understood that the smaller diameter allows for less force to be used in sealing the extended crimpable section and provides a crimped seal that is not too large when crimped. An occlusive balloon 32 is located along the distal portion 26 of guidewire assembly 22. The occlusive balloon 32 is fluidly connected via a lumen 34 to the proximal end 36 of guidewire assembly 22, with channels or holes 35 allowing for fluid communication between lumen 34 and occlusive balloon 32. In a preferred embodiment, a flexible tip 38 is positioned at the distal end 40 of distal portion 26 of the guidewire assembly 22.
Preferably, distal portion 26 of guidewire assembly 22 includes a tapered portion 42 to increase the flexibility and transition properties of the distal portion 26 of guidewire assembly 22.

Preferably, sealing system 60 is implemented as part of a handheld apparatus that also includes gas inflation/evacuation system 80. Alternatively, sealing system 60 may be a handheld unit completely separate from the gas inflation/evacuation system 80. Sealing system 60 includes a first aperture 62 into which the proximal end 36 of guidewire assembly 22 is insertable so as to operably position at least a portion of extended sealable section 28 in relation to sealing system 60. Sealing system 60 further includes a second aperture 64 that is fluidly connectable to gas inflation/evacuation system 80. The sealing system 60 includes means for selectively sealing the extended sealable section which comprises a crimping mechanism 66 and a sealing mechanism 68. A passageway 70 is defined from first aperture 62 to second aperture 64 and extends through both crimping mechanism 66 and sealing mechanism68. Preferably, at least a portion of the extended sealable section 28 is inserted into first aperture 62 a sufficient distance to engage crimping mechanism 66 and sealing mechanism 68.

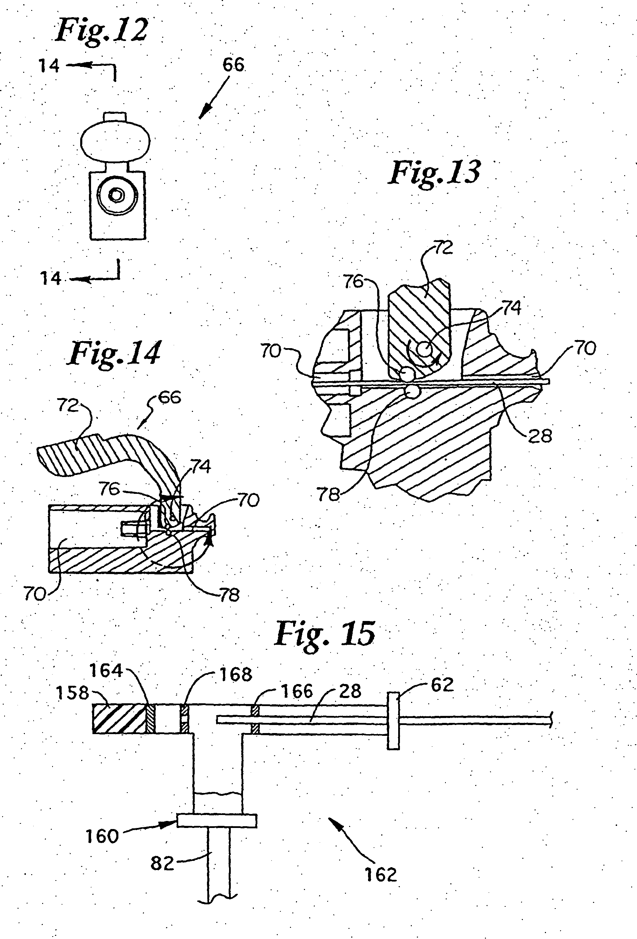

In a preferred embodiment of the crimping mechanism 66 as shown in Figs. 12-14, the crimping mechanism 66 comprises a handle 72 that actuates a pivotable cam arrangement 74 that crimps and then severs the extended sealable section 28 between a pair of rollers 76, 78 by mechanically flattening and pinching the extended sealable section 28 to the point of breaking. Preferably, the sealing mechanism 68 has a rotatable hemostatic valve positioned proximal to the crimping mechanism 66 along passageway 70. Preferably, crimping mechanism 66 and sealing mechanism 68 are arranged coaxially with each other along a straight portion of passageway 70. In this embodiment, when the proximal end 36 of guidewire assembly 22 is inserted into first aperture 62 until the proximal end 36 engages the hemostatic valve of sealing mechanism 68, the extended sealable section 28 is properly positioned relative to the crimping mechanism 66.

It will be seen that the relative distance between the engaging portions of sealing mechanism 68 and crimping mechanism 66 in this embodiment effectively defines the relative distances between a plurality of locations along extended sealable section 28 at which an airtight seal can be created, as shown in Figs. 1-2. To permit multiple inflations and deflations of the occlusive balloon 32 of the guidewire assembly 22, the length of the extended sealable section 28 should be greater than at least twice the distance between crimping mechanism 66 and sealing mechanism 68.

The gas inflation/evacuation system 80 is connected via conduit 82 to the second aperture 64 of the sealing system 60. The gas inflation/evacuation system 80 preferably includes a valve arrangement 84 that selectively couples one of an evacuation system which includes means for evacuating the guidewire assembly and an inflation system which includes means for introducing a gas into the guidewire assembly to the conduit 82. The evacuation system includes an evacuation syringe 86 which is used to evacuate the guidewire assembly 22, passageway 70, and conduit 82. The inflation system includes an inflation syringe 88 which contains a volume of a gas sufficient to inflate the occlusive balloon 32 a plurality of times. Preferably, the gas is a biocompatible gas such as carbon dioxide. Other biocompatible gasses that may be utilized with the present invention include oxygen, nitrogen, and nitrous oxide. While non-biocompatible gasses could be used, biocompatible gasses that are soluble in blood are preferred so as not to cause gas embolization in the event of a leak in the gas inflation/evacuation system. Preferably, the biocompatible gas also has a good driving gradient in addition to being soluble, in that the biocompatible gas will effectively go into a solution, in this case blood, better than ambient air. Although not preferred, low viscosity biocompatible liquids or foams also may be used for inflation provided the surface tension of the fluid is sufficient to permit the rapid inflation and deflation contemplated by the present invention. Optionally, a pressure gauge 90 can be associated with the inflation syringe 88.

It will be understood that if the guidewire assembly 22, including the occlusive balloon 32, could be verified as being capable of repeated inflations and deflations without any leakage or bursting of the occlusive balloon, then the evacuation portion of the gas inflation/evacuation system 80 would not be necessary, as the evacuation portion of the gas inflation/evacuation system 80 is intended for safety purposes to ensure that air within the guidewire assembly 22 and sealing system 60 is not introduced into the blood stream in the event of a failure, leakage or bursting of any component.

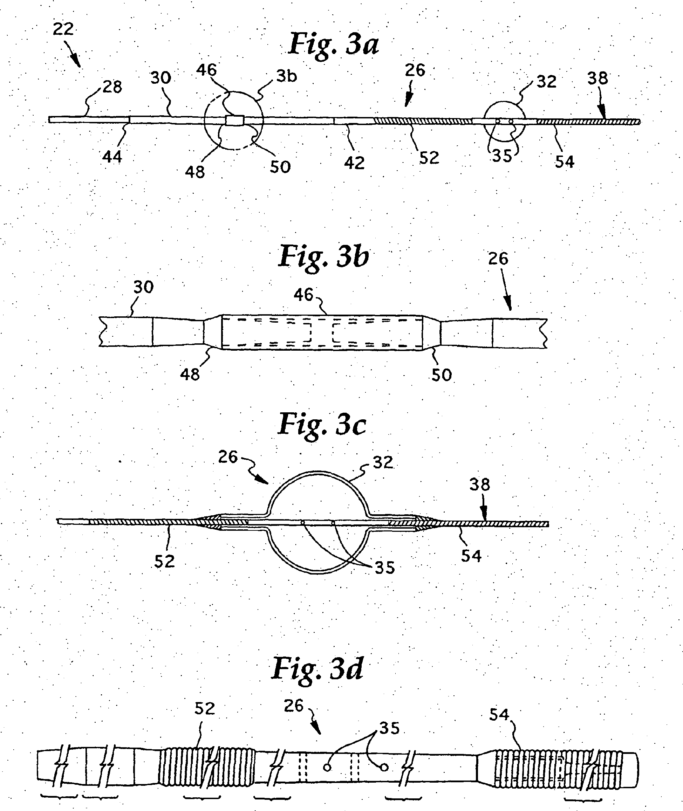

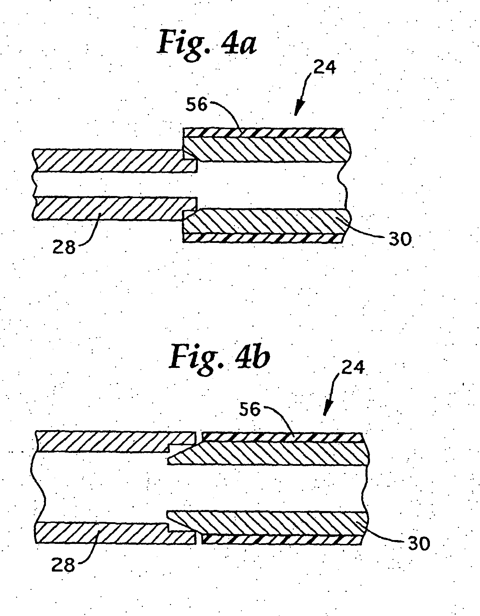

In a preferred embodiment shown in Figs. 3a-3d, 4a and 4b, the main body portion 30 of the guidewire assembly 22 is formed of a primary stainless steel hypotube having an outer diameter of 0,33mm (0.013 inch) and an inner diameter of 0,18mm (0.007 inch). To accomplish passive deflation in the desired time of less than one minute when the extended sealable section 28 is cut, it is preferable that the main body portion 30 have an inner diameter of at least 0,05mm (0.002 inch). The extended sealable section 28 of guidewire assembly 22 is comprised of a crimp tube also formed of stainless steel and having an outer diameter of 0,23mm(0.009 inch) to 0.38mm(0.015 inch) and an inner diameter of at least 0.05mm (0.002 inch) and preferably about 0,13mm (0.005 inch). The extended sealable section 28 is preferably a separate piece secured to the proximal portion 24 by a laser weld 44 (see Figs. 1, 2 and 3a) of sufficient strength. Alternatively, the extended sealable section 28 may be formed by centerless grinding or reducing the outer diameter of a portion of the proximal portion 24 of the main body portion 30 of guidewire assembly 22. Still other embodiments may enable the extended sealable section to be a modified, treated or otherwise fabricated portion of the proximal portion 24 of the main body portion 30 of guidewire assembly 22 that is suitable for the particular sealing technique to be used. As shown in Fig. 4a, in one embodiment the distal end of the extended sealable section 28 is preferably centerless ground and press fit within a chamfered proximal end of the main body portion 30. Alternatively, as shown in Fig. 4b, a chamfered crimp arrangement could be used. Still further, a separate joining/crimping tube or other tubular joining arrangements could be used. Preferably, a protective polymer coating 56 of polytetrafluoroethylene (PTFE) or a hydrophilic coating is applied by any of a number of known techniques such that the coating 56 surrounds the main body portion 30. The protective polymer coating 56 is preferably about 10µm +/- 7,6 µm (0.0004 +/- 0.0003 inch) thick such that the effective outer diameter of the main body portion 30 of guidewire assembly 22 is 0,335mm-0,365mm (0.0132 - 0.0144 inch).

In this embodiment, the extended sealable section 28 can be made of any material that when deformed and severed retains that deformation so as to form an airtight seal. When crimped and severed, it is preferable that the extended sealable section 28 not present a sharp, rigid point that is capable of piercing a gloved hand. It has been found that as long as the preferred embodiment is not gripped within less than one inch of the proximal end of the extended sealable section 28, the severed proximal end of the extended sealable section 28 does not penetrate a standard surgical glove. In addition, the extended sealable section 28 must have sufficient strength in terms of high tensile and kink resistance to permit catheter devices to repeatedly pass over the extended sealable section 28.

In this embodiment, the main body portion 30 is preferably secured to the distal portion 26 using a Ni-Ti or stainless steel sleeve 46 laser welded to the main body portion 30 at laser weld 48 and crimped to the distal portion 26 at crimp 50. The distal portion 26 is preferably formed of a Ni-Ti alloy having an inner diameter of 114µm (0.0045 inch) and an outer diameter that ranges from (0,355mm 0.014 inch) to (0,190mm 0.0075 inch) to form tapered portion 42, preferably formed by a centerless grinding process. Preferably, the distal portion includes a pair of coil sections, proximal tip coil 52 and distal tip coil 54, that are longitudinally spaced apart and adjacent to the holes 35 and that assist in providing a better surface for bonding the occlusive balloon 32 to the distal portion 26. Figs. 3c and 3d provide detailed views of this embodiment. This arrangement also tends to increase the visibility of the location of the occlusive balloon 32 under fluoroscopy, as the occlusive balloon 32 filled with a biocompatible gas will be radiotranslucent when compared to the two coils 52 and 54. Alternatively, a platinum markerband could be located around the distal portion 26 just proximal to the occlusive balloon 32 to serve as a radiopaque/MRI marker. The flexible tip 38 is a coiled tip attached to distal portion 26 distal to occlusive balloon 32, preferably by a crimp. Alternatively, a sleeve could be welded to the flexible tip 38, and the tapered portion 42 could then be inserted into this sleeve and crimped.

Alternatively, any number of other alloys or polymer materials and attachment techniques could be used in the construction of the guidewire assembly 22, provided the materials offer the flexibility and torque characteristics required for a guidewire and the attachment techniques are sufficiently strong enough and capable of making an airtight seal. These materials include, but are not limited to, Ni-Ti, 17-7 stainless steel, 304 stainless steel, cobalt superalloys, or other polymer, braided or alloy materials. The attachment techniques for constructing guidewire assembly 22 include, but are not limited to, welding, mechanical fits, adhesives, sleeve arrangements, or any combination thereof.

The occlusive balloon 32 may be made of any number of polymer or rubber materials. Preferably, the occlusive balloon is preinflated to prestretch it so that expansion is more linear with pressure. Preferably, the pressure supplied by gas inflation/evacuation system 80 is designed to stay well within the elastic limit of the occlusive balloon 32. A two-layer occlusive balloon arrangement, adding gas and/or liquid between balloon layers, may be used in an alternate embodiment to increase visibility of the distal end 40 of the distal portion 26 of the guidewire assembly 22 under fluoroscopy.

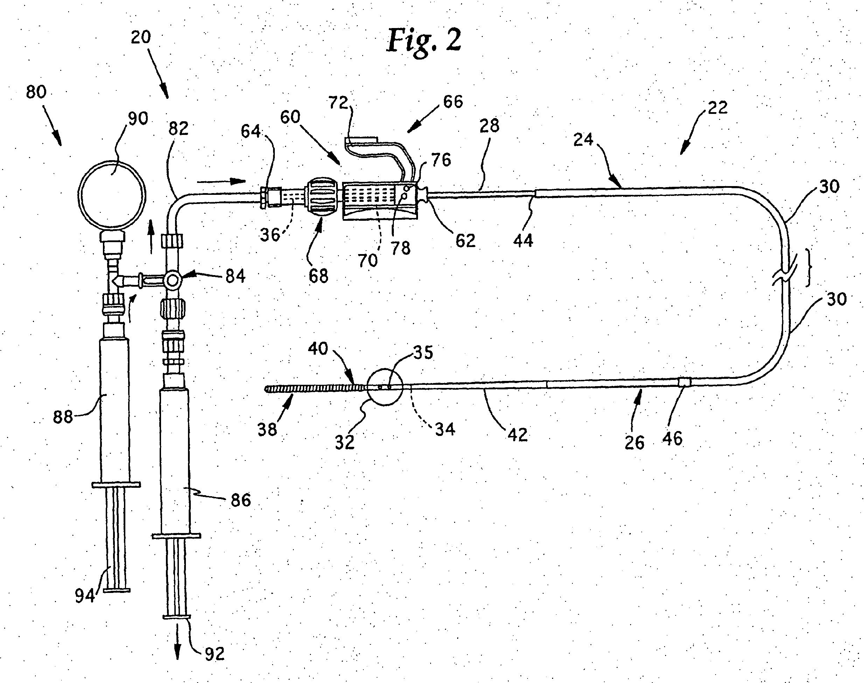

In practice, medical personnel gain entry to the vessel lumen prior to use of the guidewire occlusion system 20. The extended sealable section 28 of the proximal portion 24 of guidewire assembly 22 is inserted into first aperture 62 and connected via sealing mechanism 68. The distal portion 26 of guidewire assembly 22 is inserted into the vessel lumen, and occlusive balloon 32 is inserted to a point distal to the vessel occlusion. Valve arrangement 84 is set for evacuation. Evacuation syringe plunger 92 of evacuation syringe 86 is slidably withdrawn removing any air from guidewire assembly 22. Valve arrangement 84 is then set for inflation. Inflation syringe plunger 94 of inflation syringe 88 is slidably advanced inserting a volume of an inert gas into guidewire assembly 22. The inert gas inflates occlusive balloon 32 as shown in Fig. 2. During inflation, the medical personnel monitor pressure gauge 90 to ensure that the inflation pressure does not exceed the burst rating of the occlusive balloon 32 and to gauge the relative size of the occlusive balloon 32 as it is inflated. Following inflation of occlusive balloon 32, crimping mechanism 66 is employed to crimp the extended sealable section 28 of guidewire assembly 22, thereby sealing the guidewire assembly 22 to maintain the occlusive balloon 32 in an inflated state. Sealing mechanism 68 is released and the extended sealable section 28 is removed from first aperture 62 such that the proximal portion 24 of the guidewire assembly 22 is free of mechanical or other obstructions and can function as a conventional guidewire. When the medical personnel decide to deflate the occlusive balloon 32, the extended sealable section 28 is cut using a medical scissors or the like distal to the existing crimp in the extended sealable section 28. When the medical personnel deem reinflation of the occlusive balloon 32 to be necessary, the extended sealable section 28 of the proximal portion 24 is reinserted into first aperture 62. Sealing mechanism 68 is then reactivated and the evacuation/inflation process can be repeated. It will be understood that a crimping handle 72 may also be provided with a separate severing arrangement to cut the extended sealable section 28. Alternatively, extended sealable section 28 may be scored or otherwise weakened in selected locations to assist in crimping or severing, including severing by repeated bending back and forth at one of the scored locations. In another embodiment, the extended sealable section 28 could be broken off rather than sheared by using a brittle metal for the extended sealable section that aids in the severing of the extended sealable section 28.

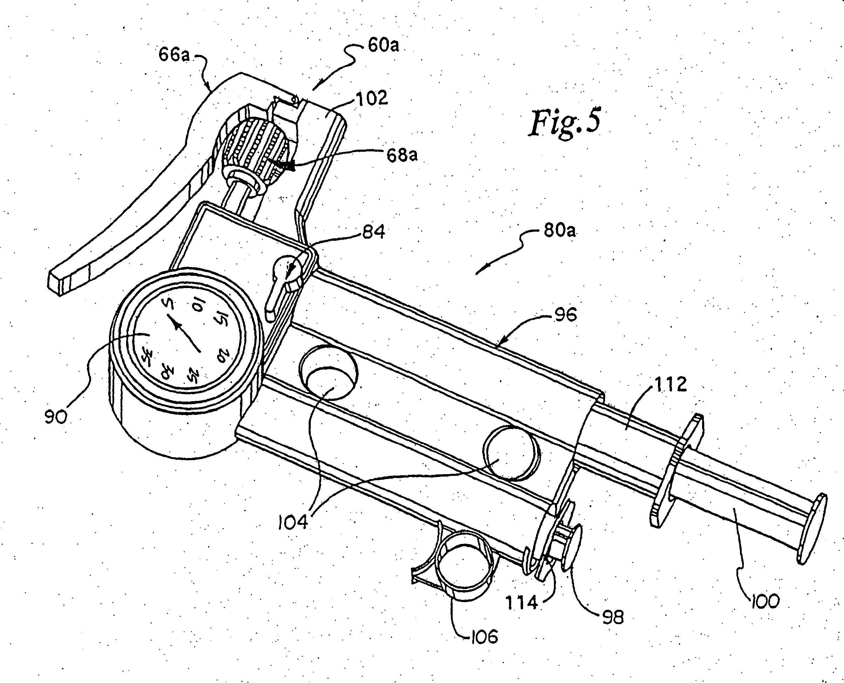

Fig. 5 shows an alternative unitized gas inflation/evacuation system 80a and also an alternative sealing system 60a. Assembly body 96 contains individual inflation syringe 114 with inflation syringe plunger 98 and individual evacuation syringe 112 with evacuation syringe plunger 100. Assembly body 96 contains pressure gauge 90. Attached to assembly body 96 is support structure 102 which supports a sealing system 60a that includes crimping mechanism 66a and sealing mechanism 68a. Valve arrangement 84 is mounted on the surface of assembly body 96. Assembly body 96 contains two fingergrip bores 104. Attached to assembly body 96 is fingergrip 106. In the preferred embodiment, the assembly body 96 is constructed of a suitable inert plastic polymer, although any polymer material used in construction of medical devices could be used. In another embodiment, the assembly body 96 can be constructed of suitable metal alloys or even of tempered glass or any combination thereof.

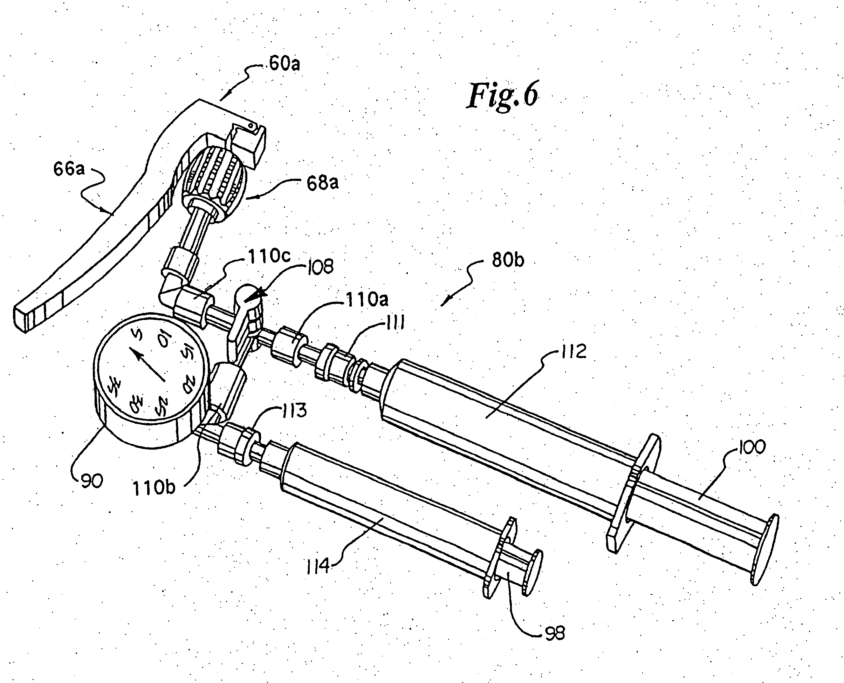

Fig. 6 shows an alternative gas inflation/evacuation system 80b in use with sealing system 60a. Valve arrangement 108 has three interconnect fittings 110a, 110b and 110c. Attached to interconnect fitting 110a is evacuation syringe 112. Evacuation syringe 112 includes evacuation syringe plunger 100. Attached to interconnect fitting 110b is pressure gauge 90. Pressure gauge 90 is fluidly interconnected to inflation syringe 114. Inflation syringe 114 includes inflation syringe plunger 98. Attached to the interconnect fitting 110c is sealing system 60a comprised of crimping mechanism 66a and sealing mechanism 68a. Preferably, one-way check valves 111 and 113 are respectively connected between interconnect fitting 110a and evacuation syringe 112 between interconnect fitting 110b and inflation syringe 114 as a safety measure to ensure only one-way flow of the gas within the gas inflation/evacuation system 80b. One-way check valve 113 ensures that only the carbon dioxide gas is delivered out of the gas inflation/evacuation system and prevents any reinfusion of air that has been evacuated from the gas inflation/evacuation system.

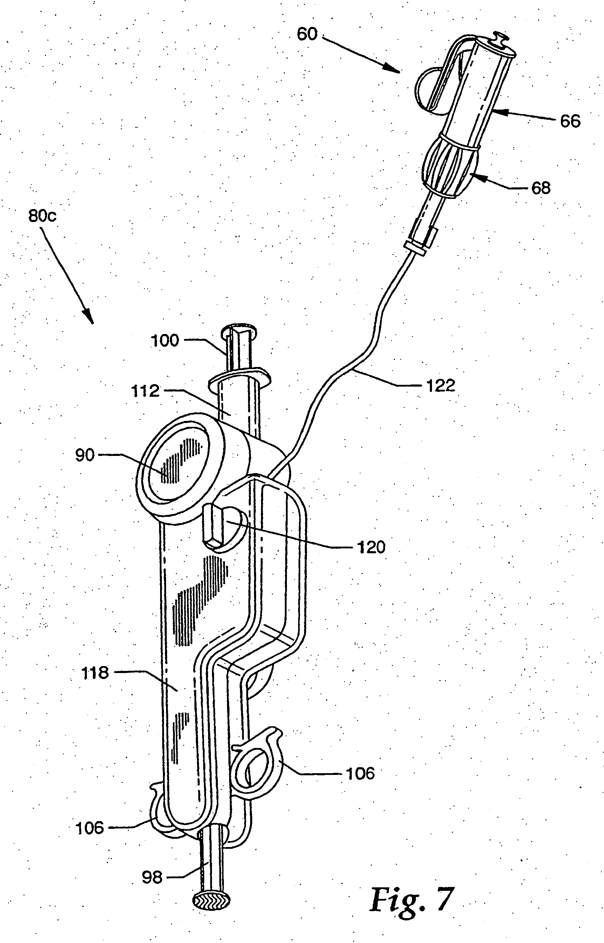

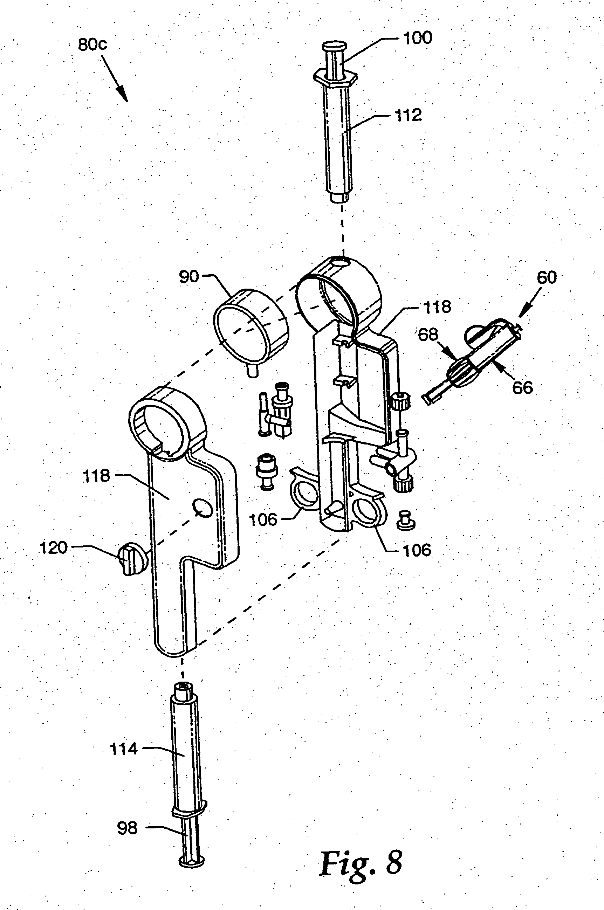

Figs. 7 and 8 show an alternative gas inflation/evacuation system 80c with sealing system 60. Assembly body 118 contains inflation syringe 114 and evacuation syringe 112. Inflation syringe 114 includes inflation syringe plunger 98. Evacuation syringe 112 includes evacuation syringe plunger 100. Knob 120 connected to valve arrangement 108 is mounted on the exterior of assembly body 118. Pressure gauge 90 is contained within assembly body 118. Assembly body 118 contains fingergrips 106. Conduit 122 is attached to assembly body 118. At the distal end of conduit 122 is sealing system 60 which is comprised of crimping mechanism 66 and sealing mechanism 68.

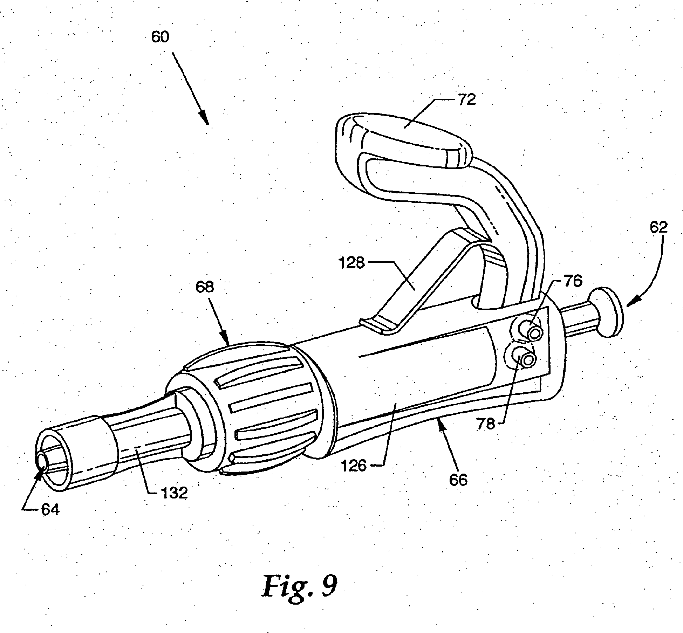

Fig. 9 shows an embodiment of the sealing system. Specifically, Fig. 9 shows sealing system 60 which is comprised of sealing mechanism 68 and crimping mechanism 66. Crimping mechanism 66 is comprised of crimp body 126, handle 72, handle return 128, and first aperture 62. Sealing mechanism 68 is comprised of sealing body 132 and second aperture 64. Sealing system 60 has a passageway 70 (see Figs. 1 and 2) fluidly interconnecting first aperture 62 and second aperture 64.

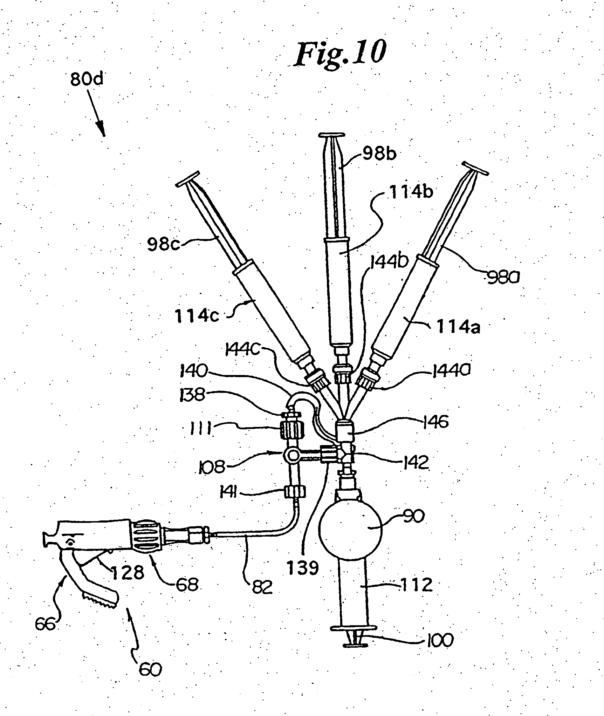

Fig. 10 shows an alternative gas inflation/evacuation assembly 80d coupled to sealing system 60. Valve arrangement 108 has a coupling 141 connected to conduit 82 and a port 138 that is attached via one-way check valve 111 and hose 140 to evacuation syringe 112. Attached to an interconnect fitting 139 of the valve arrangement 108 is inflation manifold 142. Inflation manifold 142 is connected to connector 146 and pressure gauge 90. Inflation manifold 142 has three check valves 144a, 144b and 144c. Check valves 144a, 144b and 144c are connected to respective inflation syringes 114a, 114b and 114c which have respective inflation syringe plungers 98a, 98b, and 98c. In this embodiment, evacuation syringe 112 is mounted behind pressure gauge 90. As with the other embodiments, the distal end of conduit 82 is connected to sealing system 60. Sealing system 60 is comprised of sealing mechanism 68 and crimping mechanism 66.

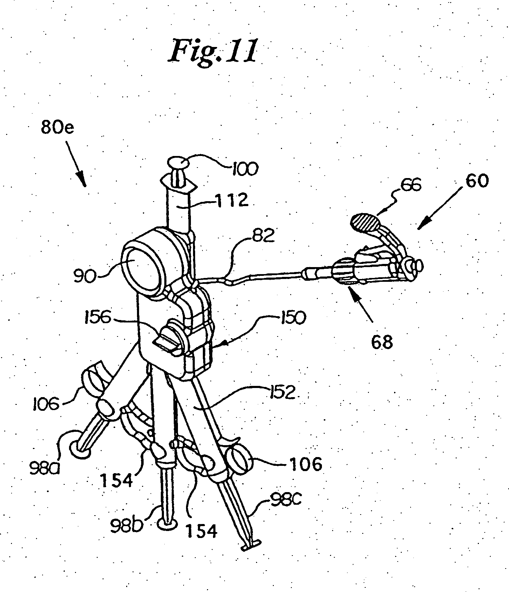

Fig. 11 shows an alternative gas inflation/evacuation system 80e that is similar to the gas inflation/evacuation system 80d shown in Figure 10 except that the components are arranged in a common housing 150. Common housing 150 has internal sealed channels that fluidly interconnect via valve arrangement 108 to evacuation syringe 112 and to inflation syringes 114a, 114b and 114c and pressure gauge 90. Common housing 150 has structure 152 that defines chambers for the three inflation syringes 114a, 114b and 114c. Common housing 150 also includes structure defining external fingergrips 106 and internal fingergrips 154 between adjacent portions of structure 152. Common housing 150 also contains structure for integrating evacuation syringe 112 and pressure gauge 90 as part of the common housing 150. An external knob 156 connects to the valve arrangement 108.

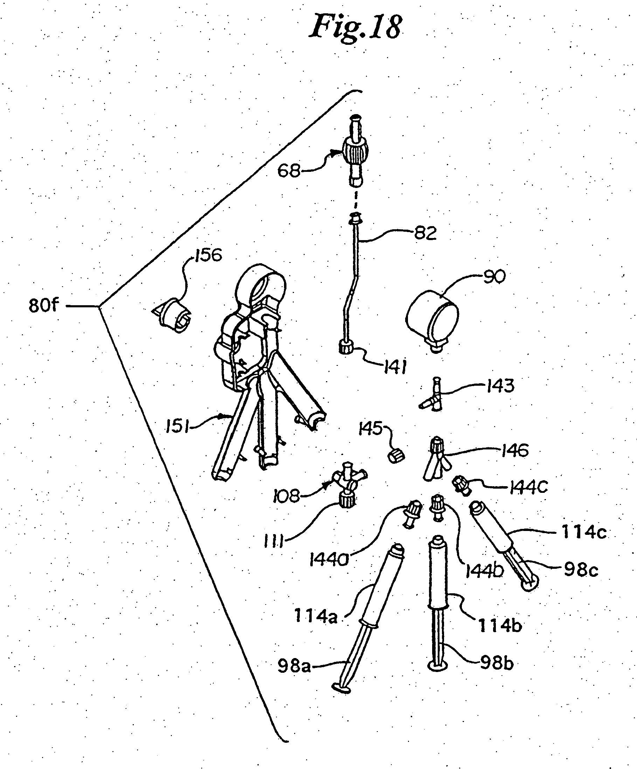

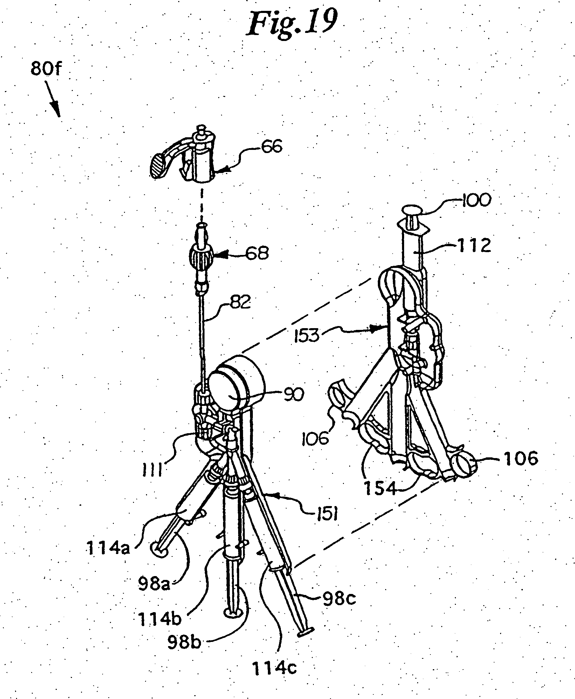

Figs. 18 and 19 show an alternative embodiment to that shown in Fig.11. Rather than utilizing the common housing 150 with internal sealed channels, an assembled gas inflation/evacuation system 80f, substantially similar to the gas inflation/evacuation system 80d shown in Fig. 10, is securely placed within a two-part housing such that the two-part housing provides a protective and functional casing around the gas inflation/evacuation system 80f. As demonstrated in the exploded view of Fig. 18, the previously described components of the gas inflation/evacuation system 80d are assembled prior to fitting of the housing. In addition to the components described above with relation to Fig. 10, this exploded view shows two additional components: namely, tee connector 143 and coupling 145. Tee connector 143 is intermediately connected to pressure gauge 90 at one end and connector 146 at the other end. Further, coupling 145 interconnects valve arrangement 108 to tee connector 143. Upon completion of the component assembly, the assembled system is securely placed within a top housing half 151, as shown in Fig 19. Once secured, a compatible bottom housing half 153, as also shown in Fig. 19, is joined with top housing half 151 to form the full housing. This joining of top housing half 151 and bottom housing half 153 can be achieved using a myriad of techniques, such as adhesive bonding, heat bonding, chemical bonding, pressure fittings, snap connectors, clip connectors, fasteners such as screws and bolts, and the like.

The embodiments shown in Figs. 10, 11, 18, and 19 allow for effective pressurization of occlusive balloon 32 at less than 2 atmospheres while reducing the total volume of gas that might be introduced into a patient in the event of a leak in the guidewire occlusion system 20. Depending upon the desired inflation pressure and the total number of inflation cycles, the total amount of pressurized gas in a single inflation syringe such as 88 in Figs. 1 and 2 or 114 in Figs. 5-8 can be significant. If a leak were to occur, the entire contents of a single inflation syringe would be susceptible to that leak. By using a separate inflation syringe 114a, 114b, 114c for each inflation in the embodiments shown in Figs. 10, 11, and 18 and 19, these alternate embodiments provide a simple way of decreasing the total amount of pressurized gas that might be introduced into a patient in the event of a leakage in the guidewire occlusion system 20.

A similar result could be achieved by manually attaching separate inflation syringes 114a, 114b, 114c and an evacuation syringe 112 directly to the sealing system 60 by way of a Luer lock or the like. While such an embodiment would not be as quick or convenient as the preferred embodiment, this alternative would eliminate the volume of gas required for the conduit 82 and within common housing 150, as well as the need for a valve arrangement 108.

In alternate examples that do not form part of the invention, the sealing system could include means for selectively sealing involving techniques other than crimping to accomplish multiple airtight seals along the course of the extended sealable section 28. One alternate example, as portrayed in Figure 15, would involve the insertion of some form of sealant material 158 into the proximal end of the extended sealable section 28, such as wax, plastic, polymer or metal inserts or plugs. Conduit 82 is attached to sealing mechanism 162 through the conduit aperture 160. In this example, sealant material 158 is confined by sealant confinement layer 164 residing within sealing mechanism 162. Preferably for this example, sealant material 158 is a wax or gel that is flowable at higher temperatures and might be melted during sterilization of the sealing system. Sealant confinement layer 164 is a foil layer or thin layer of non-meltable material capable of confining a flowable material during any sterilization process or exposure to higher temperature. The proximal end of extended sealable section 28 is inserted through first aperture 62 until it is past operational O-ring 166 or some other form of sealable/deformable material such as a silicone puncture seal or similar membrane seal. When it is desired to seal the extended sealable section 28, the extended sealable section 28 is further inserted past a sealant O-ring 168, then through sealant confinement layer 164, and finally into sealant material 158. Sealant material 158 is deposited in the proximal end of extended sealable section 28, thus preventing the guidewire assembly 22 from being evacuated. Extended sealable section 28 can then be slidably withdrawn through the sealant o-ring 168, through the operational O-ring 166, and through the first aperture 62, thereby effectively disengaging the guidewire assembly 22 from the sealing mechanism 162. The o-rings 166 and 168 serve as wiping structures to remove excess sealant material from the outside of the extended sealable section 28. Other alternate examples involve heating the extended sealable section 28 when it is formed of metal or polymer material so as to create a constriction, or applying electrical or magnetic energy to arc or weld material within the extended sealable section 28 to create a constriction. In one example, the equivalent of a spot welder could be used in place of the crimping mechanism 66 to accomplish the same purpose of sealing, and then severing the extended sealable section 28. Alternative examples could use other sealing techniques to seal the guidewire assembly 22. These methods could include, but are not limited to, ones utilizing a heat source to melt the extended sealable section, ones using a heat source to apply a glue or gel, methods involving insertion of a plug material, methods using magnetics to manipulate a sealing material, or methods utilizing small occlusive devices.

Depending on the sealing method specified different deflation techniques can be utilized. For the present example, the extended sealable section 28 is of sufficient length to allow deflation through the shearing, breaking or opening of the extended sealable section 28 distal to the sealant material 158 located in the proximal end of the extended sealable section 28. By having sufficient length of the extended sealable section 28, the guidewire assembly 22 can be coupled to the gas inflation/evacuation system 80 (or 80a-80f) multiple times, allowing the occlusive balloon 32 to be inflated and deflated multiple times. other examples will use methods of deflation including melting the sealant material 158, removing a plug of sealant material 158, and various other methods not requiring the extended sealable section 28 to be sheared.

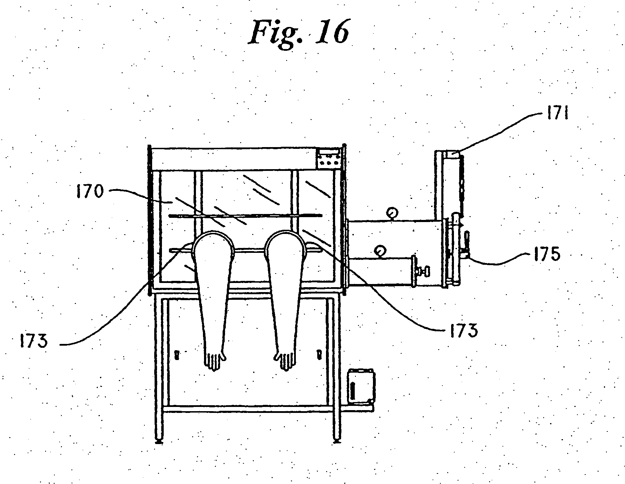

In one embodiment, the guidewire occlusion system 20 is preferably pre-assembled and packaged in an environment consisting of an appropriate biocompatible gas. Fig. 16 shows equipment with which the guidewire occlusion system 20 is assembled and packaged. The guidewire occlusion system 20 is assembled and packaged in a sealed chamber 170. Sealed chamber 170 is equipped with a venting duct 171, sealed handling ports 173, and an atmosphere control system 175. The venting duct 171 and atmosphere control system 175 provide the overall system for maintaining a biocompatible gas atmosphere within the sealed chamber 170. Sensory readings within the sealed chamber 170 provide the atmosphere control system 175 with the data needed to adjust the biocompatible gas levels within the sealed chamber 170. Stored biocompatible gas is introduced into the sealed chamber 170 through the venting duct 171. Assembling and packaging of the guidewire occlusion system 20 and/or any of the pre-assembled components is achieved with the use of the sealed handling ports 173. The ports 173 are sterilized and sealed so that an assembler or packager positioned outside the sealed chamber 170 can access the contents of the chamber without introducing contamination through actual human contact or through the introduction of undesirable gases and airborne contaminants. These ports 173 could be constructed of flexible glove-like attachments, as shown, or they could be robotic devices operable within the sealed chamber 170 through controls external to the sealed chamber 170. The equipment could be two or more sealed chambers.

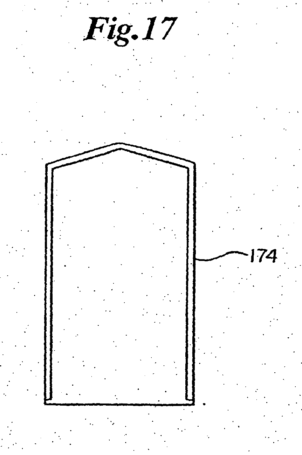

After a guidewire assembly 22, a sealing system 60 (or 60a) and a gas inflation/evacuation system 80 (or 80a-80f) are placed in a sealed chamber 170, they are assembled to form the guidewire occlusion system 20 and placed into biocompatible packaging 174 (Fig. 17). Biocompatible packaging 174 is hermetically sealed so that the internal volume of both biocompatible packaging 174 and guidewire occlusion system 20 is composed solely of biocompatible gas. A preferred embodiment of the biocompatible packaging 174 is shown in Fig. 17. The biocompatible packaging 174 is preferably in the form of a foil pouch. This foil pouch is made from a medical packaging film with the following laminates: an 8.75 micron foil layer, an adhesive layer, a white polyethylene layer, and a 12 micron PET layer. The foil pouch has a preferred total thickness of approximately 3.6 millimeters, and a minimum bond strength of 4,45N (one pound). In addition, the preferred barrier properties of the film will be an oxygen transmission 155µL/m²/24hr(0.1cc/100sq.in/24 hr). 22,8° Celsius (73 degrees F., 0% RH) ASTM 3985 and moisture vapor transmission 0,155gm H20/m²/24hr (.01gm H20/100sq.in/24hr). 37,8° Celsius (100 degrees F., 90%RH) ASTM F1249. It will be understood by those skilled in the art that this biocompatible foil pouch can be contained and/or attached within an outer packaging or container, such as a cardboard box, a plastic container, or the like. Such an outer packaging will facilitate shipping, labeling, storage, and handling of the biocompatible packaging 174 and its contents.

In practice, medical personnel gain access to the vessel lumen through which the guidewire assembly 22 will travel. The guidewire occlusion system 20 is removed from biocompatible packaging 174. Flexible tip 38 is inserted in the vessel lumen and is manipulated to a point beyond the vessel occlusion. Valve arrangement 84 (or 108) is adjusted to the evacuation position and evacuation syringe plunger 92 (or 100) is slidably withdrawn to remove any gas present in the guidewire assembly 22. Valve arrangement 84 (or 108) is then adjusted to the inflation position and inflation syringe plunger 94 (or 98, 98a, 98b, 98c) is slidably inserted causing occlusive balloon 32 to inflate.

Following inflation of occlusive balloon 32, handle 72 of the crimping mechanism 66 (or the handle of 66a) is depressed causing roller 76 and roller 78 to crimp and preferably sever the extended sealable section 28 of guidewire assembly 22. Severing of the extended sealable section 28 serves as an immediate verification of the creation of an effective seal. Sealing mechanism 68 (or 68a) can be released and guidewire assembly 22 can be completely removed from the sealing system 60 (or 60a) allowing the occlusive balloon 32 to remain inflated while occlusive substance treatment occurs. Following treatment, the extended sealable section 28 can be sheared or broken off, resulting in the deflation of the occlusive balloon 32. If occlusive treatment is complete, guidewire assembly 22 can be removed from the vessel lumen. If additional treatment is required, extended sealable section 28 can be reattached to sealing system 60 (or 60a) through first aperture 62. Sealing mechanism 68 (or 68a) can be retightened and the evacuation/inflation process can be repeated.

In a preferred embodiment of the present invention, the guidewire assembly 22 is utilized as the guidewire for an atherectomy or thrombectomy procedure of the type described in U.S. Patent Nos. 5,370,609 or 5,496,267.

In each of these procedures, the guidewire assembly 22 is introduced into the patient, the occlusive balloon 32 is inflated, and then the atherectomy or thrombectomy catheter arrangement is slid over the proximal end 36 of the guidewire assembly 22 and advanced until it is proximate and proximal to the location of the occlusive balloon. The procedure is performed for a time period consistent with the desired maximum length for blockage of the particular vessel, typically within 5 minutes, at which time the extended sealable section 28 of the guidewire assembly 22 may be severed to deflate the occlusive balloon 32, thereby reestablishing blood flow within the vessel. Depending upon the nature of the procedure, the catheter arrangement may be removed from the vessel or left in place. Preferably, an evacuation of any plaque material or other debris dislodged by the therapy is accomplished before deflation of the occlusive balloon 32. The occlusive balloon 32 is reinflated prior to reinitiation of the procedure.

It will be understood that because gas is used as the inflation medium instead of liquid, the wall thickness and therefore the stiffness of tubular members of the guidewire assembly 22 can be increased to effectively match the stiffness and flexibility of an ideal solid guidewire. Stiffness increase is dramatic as a result because stiffness of the tube is governed by the equation (R(o)**4-R(i)**4), such that an increase in wall thickness effectively quadruples the increase in stiffness of the guidewire assembly.

Rapid inflation and deflation of an occlusive balloon is the key to a successful occlusion device. The viscosity of the inflation fluid and resistance through the evacuation/inflation lumens dictate the effective speed of inflation and deflation. By lowering the viscosity of the inflation fluid, the present invention is able to increase the amount of resistance through the evacuation/ inflation lumen that can be overcome. This results in being able to use a smaller inner diameter tube for the evacuation/ inflation lumen which allows for a significant increase in the structural robustness of the guidewire assembly while maintaining the desired inflation and deflation properties. The increase in allowable resistance also allows for the use of longer guidewire assemblies, specifically guidewire assemblies that are a more typical exchange length. With typical high viscosity inflation fluids used to inflate liquid occlusive balloons, for example, it is not practical to develop an exchange length guidewire assembly because of the long deflation times associated with evacuation of the high viscosity inflation fluid through a much longer lumen.

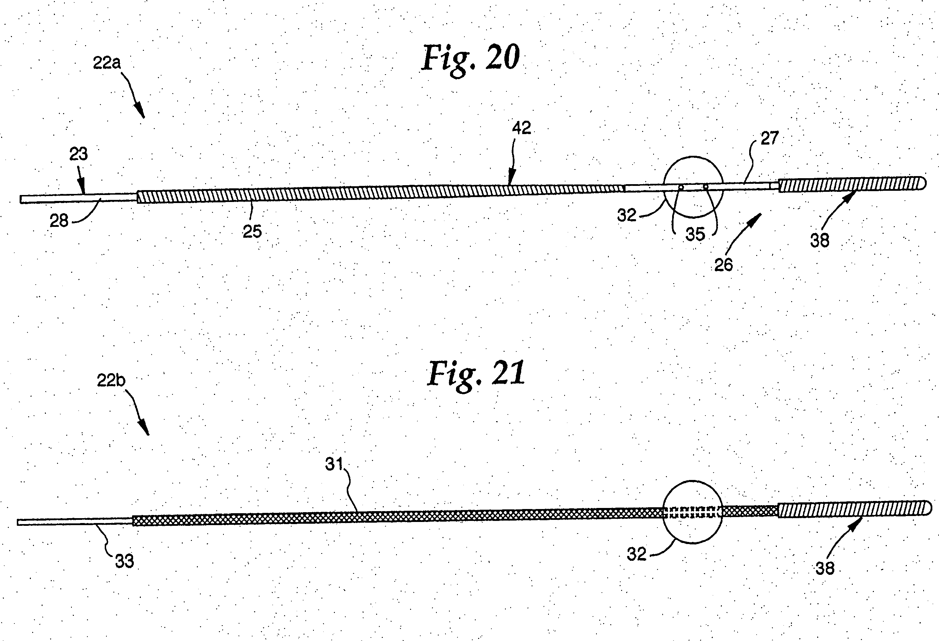

Another guidewire assembly embodiment is shown in Fig. 20 and is designated 22a. The guidewire assembly 22a includes a continuous stainless steel tube 23 of 0,18mm(0.007 inch) outer diameter by 0,10mm (0.004 inch) inner diameter having a wire 25 of 0,076mm (0.003 inch) diameter wrapped therearound except for along the extended sealable section 28 and, optionally, except for along a section 27 at the distal portion 26 of the continuous stainless steel tube 23. The distal portion 26 distal of the occlusive balloon 32 could be pinched in a taper to provide the desired flexible tip 38. One or more holes 35 are provided in the stainless steel tube 23 along section 27 over which the occlusive balloon 32 is positioned and adhesively secured. This embodiment would be less expensive by avoiding the use of Ni-Ti alloy and eliminating the need for laser welding or other attachment techniques along the guidewire assembly 22a.

Another guidewire assembly embodiment is shown in Fig. 21 and is designated 22b. The guidewire assembly 22b includes a braided polyimide hypotube 31 having a tapered Ni-Ti core rod 33 positioned therein. The tapered Ni-Ti core rod 33 provides stiffness to the guidewire assembly 22b, but is much less expensive than a Ni-Ti tube. At least a proximal portion of the tapered Ni-Ti core rod 33 is tubular having a lumen defined therein and extending beyond a proximal end of the braided polyimide hypotube 31 to define the extended sealable section. Alternatively, the braided polyimide hypotube 31 could include composite carbon fiber reinforcement to increase the strength thereof. Numerous arrangements for securing the tapered Ni-Ti core rod 33 within the braided polyimide hypotube 31 and for attaching the flexible tip 38 and the occlusive balloon 32 to the braided polyimide hypotube 31 are available with respect to this embodiment.

The present invention may be embodied in other specific forms without departing from the essential attributes thereof; therefore, the illustrated embodiments should be considered in all respects as illustrative and not restrictive, reference being made to the appended claims rather than to the foregoing description to indicate the scope of the invention.

| **GUIDEWIRE OCCLUSION SYSTEM UTILIZING REPEATABLY INFLATABLE GAS-FILLED OCCLUSIVE DEVICE** | | | |
|---|---|---|---|
| **PARTS LIST** | | | |
| 20 | guidewire occlusion system | 46 | Ni-Ti or stainless steel |
| 22 | guidewire assembly | 48 | sleeve laser weld |
| 22a-b | guidewire assemblies | 50 | crimp |
| 23 | stainless steel tube | 52 | proximal tip coil |
| 24 | proximal portion | 54 | distal tip coil |
| | | 55 | crimp |
| 25 | wire | 56 | protective |
| 26 | distal portion | | polymer coating |
| 27 | section | 60 | sealing system |
| | | 60a | sealing system |
| 28 | extended crimpable section | 62 | first aperture |
| 30 | main body portion | 64 | second aperture |
| 31 | braided polyimide hypotube | 66 | crimping mechanism |
| 32 | occlusive balloon | 66a | crimping mechanism |
| 33 | tapered Ni-Ti core rod core rod | 68 | sealing mechanism |
| | | 68a | sealing mechanism |
| 34 | lumen | 70 | passageway |
| 35 | channel or hole | 72 | handle |
| 36 | proximal end | | |
| 38 | flexible tip | 74 | pivotable cam arrangement |
| 40 | distal end | 76 | roller |
| 42 | tapered portion | 78 | roller |
| 44 | laser weld | 80 | gas inflation/ evacuation system |
| 80a-f | gas inflation/ evacuation systems | 114a-c | inflation syringes |
| 82 | conduit | 118 | assembly body |
| 84 | valve arrangement | 120 | knob |
| | | 122 | conduit |
| 86 | evacuation syringe | 126 | crimp body |
| 88 | inflation syringe | 128 | handle return |
| 90 | pressure gauge | 132 | sealing body |
| 92 | evacuation syringe plunger | 138 | port |
| 94 | inflation syringe plunger | 139 | interconnect fitting |
| 96 | assembly body | 140 | hose |
| 98 | inflation syringe plunger | 141 | coupling |
| 98a-c | inflation syringe plungers | 142 | inflation manifold |
| | | 143 | tee connector |
| 100 | evacuation syringe plunger | 144a-c | check valves |
| 102 | support structure | 145 | coupling |
| 104 | fingergrip bore | 146 | connector |
| 106 | fingergrip | 150 | common housing |
| 108 | valve arrangement | 151 | top housing half |
| 110a-c | interconnect fittings | 152 | structure |
| 111 | one-way check valve | 153 | bottom housing half |
| | | 154 | fingergrip |
| 112 | evacuation syringe | 156 | knob |
| 113 | one-way check valve | 158 | sealant material |
| 114 | inflation syringe | 160 | conduit aperture |
| 162 | sealing mechanism | | |
| 164 | sealant confinement layer | | |
| 166 | operational O-ring | | |
| 168 | sealant O-ring | | |
| 170 | sealed chamber | | |
| 171 | venting duct | | |
| 173 | sealed handling port | | |
| 174 | biocompatible packaging | | |
| 175 | atmosphere control system | | |

## Claims

1. A guidewire occlusion system (20) for use in vascular procedures comprising:
(a) a tubular guidewire assembly having an occlusive balloon (32) proximate a distal end (26) and an extended sealable section (24) proximate a proximal end;
(b) a gas inflation/evacuation system (80) removably connectible to the proximal end of the tubular guidewire assembly, including:
(1) means for evacuating the tubular guidewire assembly; and,
(2) means for introducing a gas into the tubular guidewire assembly to inflate the occlusive balloon a plurality of times; and,
(c) a scaling system (68) removably connectible to the proximal end (36) of the tubular guidewire assembly, including:
(1) means for selectively sealing the tubular guidewire assembly by forming successive airtight seals at separate locations along the extended sealable section to retain the gas in the occlusive balloon a plurality of times, wherein the diameter of the extended sealable section when sealed is no greater than the diameter of any other portion of the tubular guidewire assembly so that the tubular guidewire assembly may be used as a guidewire to introduce a catheter over the guidewire while gas is retained in the occlusive balloon;
**characterised in that** the extended scalable section consists of an extended crimpable section (28) and the means for selectively sealing comprises a crimping mechanism (66) which deforms and crimps the extended crimpable section so as to form an airtight seal

2. The system of claim 1 wherein the tubular guidewire assembly has an effective length of at least 40 cm and an outer diameter of less than 0.02 cm (0.06 inch), wherein the extended sealable section has an effective length of at least 1 cm and an outer diameter of less than 0.13 cm (0.05 inch), and wherein the occlusive balloon can be deflated in less than two minutes.

3. The system of claim 1 wherein the tubular guidewire assembly has an effective length of at least 100 cm and an outcr diameter of less than 0.05 cm (0.018 inch), wherein the extended sealable section has an effective length of at least 5 cm and an outer diameter of less than 0.03 cm (0.012 inch), and wherein the occlusive balloon can be deflated in less than one minute.

4. The system of any preceding claim wherein the extended crimpable section is dimensioned and the crimping mechanism is arranged such that an effective outer diameter of the extended crimpable section at the location of a seal is no greater than the outer diameter of a main body portion of the tubular guidewire assembly when the extended crimpable section is crimped at the location of the seal.

5. The system of any preceding claim wherein the occlusive balloon is capable of repeated inflation and deflation during a vascular procedure in between which the proximal end of the tubular guidewire assembly is free of mechanical connections and obstructions, thereby enabling the tubular guidewire assembly to function as a conventional guidewire for catheter exchanges.

6. The system of any preceding claim further comprising means for selectively opening the extended scalable section distal to each successive permanent airtight seal to deflate the occlusive balloon.

7. The system of any preceding claim, including
(1) a handheld unit including a crimping mechanism having a first aperture and a sealing mechanism having a second aperture, there being a passageway extending from the first aperture to the second aperture for receiving the proximal end of the tubular guidewire assembly;
(2) an evacuation syringe system;
(3) an inflation syringe system containing a volume of a gas sufficient to inflate the occlusive balloon a plurality of times; and,
(4) conduits operably connecting the evacuation syringe system and the inflation syringe system to the second aperture of the handheld unit, the conduits including a valve arrangement that selectively connects only one of the evacuation syringe system and the inflation syringe system to the second aperture at a time.

8. The system of claim 8 wherein the inflation syringe system includes a plurality of individual syringes, each individual syringe containing a sufficient volume of gas to inflate the occlusive balloon one time.

9. The system of claim 8 or 9 wherein the conduit connecting to the evacuation syringe system includes a one-way check valve permitting only evacuation and the conduit connecting to the inflation syringe system includes a one-way check valve permitting only inflation.

10. The system of any preceding claim wherein the tubular guidewire member includes a main body portion formed of a stainless steel hypotube.

11. The system of claim 11 wherein the extended crimpable section is collinearly attached to the proximal end of the stainless steel hypotube and is formed of a stainless steel hypotube having an outer diameter smaller than the outer diameter of the stainless steel hypotube of the main body portion.

12. The system of claim 11 or 12 wherein the tubular guidewire member further includes a distal portion formed of a Ni-Ti alloy.

13. The system of any preceding claim wherein an extended crimpable section is weakened by scores at a plurality of locations along the extended crimpable section to aid in severing the extended crimpable section at such locations.

14. The system of any preceding claim wherein the tubular guidewire member and the extended crimpable section are formed of a continuous stainless steel tube having a wire wrapped therearound except for along the extended crimpable section.

15. The system of any preceding claim wherein the tubular guidewire member comprises a braided polyimide hypotube having a tapered core rod positioned therein.

16. The system of claim 16 wherein at least a proximal portion of the tapered core rod is tubular having a lumen defined therein and extending proximal a proximal end of the braided polyimide hypotube to define the extended crimpable section.

17. The system of any preceding claim further comprising a pair of coils positioned along a distal portion of the tubular guidewire member that are longitudinally spaced apart and adjacent to at least one hole in the tubular guidewire member that provides fluid access between the lumen and the occlusive balloon, with at least a portion of the occlusive balloon being bonded to at least a portion of each of the coils.

18. The system of any preceding claim wherein the crimping mechanism comprises:
a first roller and a second roller proximately spaced from the first roller for traversal of the proximal portion of the guidewire assembly, the first roller being connected to a handle with a pivotable cam arrangement such that force on the handle causes the first roller to proportionately approach the second roller, a first threshold force on the handle causing scaling of the proximal portion of the guidewire assembly, and a second threshold force on the handle causing severing of the proximal portion of the guidewire assembly.

19. The system or claim 19 wherein the handle is spring biased for an automatic return to an open starting position upon the cessation of a force.

## Patentansprüche

1. Ein Führungsdrahtokklusionsssystem (20) zur Verwendung in vaskularen Verfahren, aufweisend:
(a) eine schlauchförmige Führungsdrahtbaugruppe mit einem Okklusionssballon (32) in der Nähe eines entfernten Endes (26), und einem lang gestreckten abdichtbaren Abschnitt (24) in der Nähe eines nahe gelegenen Endes;
(b) ein an dem nahe gelegenen Ende der schlauchförmigen Führungsdrahtbaugruppe abnehmbar verbindbares Gasbefüll- und -entleersystem (80), enhaltend:
(1) Mittel zum Entleeren der schlauchförmigen Führungsdrahtbaugruppe, und
(2) Mittel zum Zufuhren von Gas in die schlauchförmige Fuhrungsdrahtbaugruppe, um den Okklusionsballon mehrmals aufzublasen; und
(c) ein an das nahe gelegene Ende (36) der schlauchförmigen Fuhrungsdrahtbaugruppe abnehmbar verbindbares Dichtsystem (68), enthaltend:
(1) Mittel zum selektiven Abdichten der schlauchförmigen Führungsdrahtbaugruppe durch Ausbilden von aufeinander folgenden luftdichten Abdichtungen an einzelnen Stellen entlang des langgestreckten abdichtbaren Abschnitts, um das Gas mehrmals in den Okklusionsballon einzuschließen, wobei der Durchmesser des abgedichteten langgestreckten abdichtbaren Abschnitts im abgedichteten Zustand nicht großer als der Durchmesser irgendeines anderen Abschnitts der schlauchformigen Führungsdrahtbaugruppe ist, so dass die schlauchformige Fuhrungsdrahtbaugruppe als Führungsdraht zum Einführen eines Katheters über den Fuhrungsdraht verwendet werden kann, während Gas in den Okklusionsballon eingeschlossen ist,
**dadurch gekennzeichnet, dass** der langgestreckte abdichtbare Abschnitt aus einem langgestreckten quetschbaren Abschnitt (28) besteht, und die Mittel zum selektiven Abdichten einen Quetschmechanismus (66) aufweisen, der den langgestreckten quetschbaren Abschnitt verformt und quetscht, so dass eine luftdichte Abdichtung gebildet wird.

2. System gemäß Anspruch 1, wobei die schlauchformige Führungsdrahtbaugruppe eine effektive Lange von mindestens 40 cm und einen äußeren Durchmesser von weniger als 0,02 cm (0,06 Inch) aufweist, wobei der langgestreckte abdichtbare Abschnitt eine effektive Länge von mindestens 1 cm und einen äußeren Durchmesser von weniger als 0,13 cm (0,05 Inch) aufweist, und wobei der Okklusionsballon in weniger als zwei Minuten entleert werden kann.

3. System gemäß Anspruch 1, wobei die schlauchförmige Fuhrungsdrahtbaugruppe eine effektive Länge von mindestens 100 cm und einen äußeren Durchmesser von weniger als 0,05 cm (0,018 Inch) aufweist, wobei der langgestreckte abdichtbare Abschnitt eine effektive Lange von mindestens 5 cm und einen äußeren Durchmesser von weniger als 0,03 cm (0,012 Inch) aufweist, und wobei der Okklusionsballon in weniger als einer Minute entleert werden kann.

4. System gemaß einem der vorangehenden Anspruche, wobei der langgestreckte quetschbare Abschnitt so dimensioniert und die Quetschvorrichtung so angeordnet ist, dass ein effektiver außerer Durchmesser des langgestreckten quetschbaren Abschnitts an der Stelle einer Abdichtung nicht größer als der äußere Durchmesser eines Hauptkorperabschnitts der schlauchformigen Fuhrungsdrahtbaugruppe ist, wenn der langgestreckte quetschbare Abschnitt an der Stelle der Abdichtung gequetscht wird.

5. System gemäß einem der vorangehenden Anspruche, wobei der Okklusionsballon während einem vaskularem Verfahren, unter welchem das nahe gelegene Ende der schlauchförmigen Führungsdrahtbaugruppe frei von mechanischen Verbindungen und Hindernissen ist, wiederholt befüllt und entleert werden kann, und es dabei ermoglicht, dass die schlauchförmige Führungsdrahtbaugruppe wie ein konventioneller Führungsdraht fur Katheterwechsel funktioniert.

6. System gemäß einem der vorangehenden Ansprüche, weiterhin Mittel zum selektiven Öffnen des langgestreckten abdichtbaren Abschnitts entfernt von jedem aufeinander folgenden luftdichten Verschluß aufweisend, um den Okklusionsballon zu entleeren.

7. System gemaß einem der vorangehenden Ansprüche, einschließlich
(1) einer handgehaltenen Einheit einschließlich einem Quetschmechanismus mit einer ersten Öffnung, und einem Abdichtmechanismus mit einer zweiten Öffnung, wo ein Durchgang, der sich von der ersten Öffnung zu der zweiten Öffnung zum Aufnehmen des nahe gelegenen Endes der schlauchförmigen Fuhrungsdrahtbaugruppe erstreckt, vorhanden ist,
(2) einem Entleerungsspritzensystem,
(3) einem Befüllspritzensystem, das ein Gasvolumen enthält, das ausreichend ist, den Okklusionsballon mehrmals zu befullen,
(4) Leitungen, die das Entleerungsspritzensystem und das Befullspritzensystem mit der zweiten Öffnung der handgehaltenen Einheit betreibbar verbinden, wobei die Leitungen eine Ventilanordnung umfassen, die selektiv zur gleichen Zeit nur eines des Entleerungsspritzensystems und Befüllspritzensystems mit der zweiten Öffnung verbindet.

8. System gemäß Anspruch 8, wobei das Befullspritzensystem mehrere einzelne Spritzen enthält, und jede einzelne Spritze ein ausreichendes Gasvolumen enthalt, um den Verschlussballon ein Mal zu befullen.

9. System gemäß Anspruch 8 oder 9, wobei die Leitung zur Verbindung mit dem Entleerungsspritzensystem ein Einwegrückschlagventil enthält, das nur die Entleerung erlaubt, und die Leitung zur Verbindung mit dem Befüllspritzensystem ein Einwegrückschlagventil enthält, das nur das Befüllen erlaubt.

10. System gemaß einem der vorangehenden Ansprüche, wobei das schlauchförmige Fuhrungsdrahtelement einen Hauptkörperabschnitt aus einem rostfreien Stahl-Hypotube enthält.

11. System gemäß Anspruch 11, wobei der langgestreckte quetschbare Abschnitt kollinear an dem nahe gelegenen Ende des rostfreien Stahl-Hypotube angebracht ist, und aus einem rostfreien Stahl-Hypotube gebildet wird, das einen äußeren Durchmesser hat, der geringer als der äußere Durchmesser des rostfreien Stahl-Hypotubes des Hauptkörperabschnitts ist.

12. System gemäß Anspruch 11 oder 12, wobei das schlauchformige Fuhrungsdrahtelement einen aus einer Ni-Ti-Legierung gebildeten entfernten Abschnitt enthält.

13. System gemäß einem der vorangehenden Anspruche, wobei ein langgestreckter quetschbarer Abschnitt an mehreren Stellen entlang des langgestreckten quetschbaren Abschnitts durch Kerben geschwächt ist, um das Abtrennen des langgestreckten quetschbaren Abschnitts an diesen Stellen zu unterstützen.

14. System gemäß einem der vorangehenden Ansprüche, wobei das schlauchförmige Fuhrungsdrahtelement und der langgestreckte quetschbare Abschnitt aus einem durchgehenden rostfreien Stahlrohr mit einen Draht, der, außer entlang des langgestreckten quetschbaren Abschnitts, darum gewickelt ist, gebildet werden.

15. System gemäß einem der vorangehenden Ansprüche, wobei das schlauchförmige Fuhrungsdrahtelement ein geflochtenes Polyimid-Hypotube mit einem darin angeordneten angespitzten Kernstab aufweist.

16. System gemäß Anspruch 16 wobei zumindest ein nahe gelegener Bereich des angespitzen Kernstabs rohrförmig mit einem darin definierten Lumen ist, und sich in der Nähe des nahe gelegenen Endes des geflochtenen Polyimid-Hypotubes erstreckt, um den langgestreckten quetschbaren Abschnitt zu definieren.

17. System gemäß einem der vorangehenden Ansprüche, weiterhin ein Paar entlang eines entfernten Abschnitts des schlauchförmigen Führungsdrahtelements liegende Wendel aufweisend, die in Langsrichtung voneinander beabstandet und benachbart zu zumindest einem Loch in dem schlauchförmigen Führungsdrahtelement sind, das einen Flüssigkeitszugang zwischen dem Lumen und dem Okklusionsballon bereitstellt, wobei zumindest ein Abschnitt des Okklusionsballons mit zumindest einem Abschnitt jeder der Wendel verbunden bzw. verklebt ist.

18. System gemaß einem der vorangehenden Anspruche, wobei der Quetschmechanismus aufweist:
eine erste Rolle und eine benachbart zur ersten Rolle beabstandete zweite Rolle zum quer hindurchlaufen des nahe liegenden Abschnitts der Führungsdrahtbaugruppe, wobei die erste Rolle mit einem Handgriff mit einer schwenkbaren Nockenanordnung verbunden ist, so dass eine Kraft auf den Handgriff die erste Rolle dazu veranlasst, sich proportional der zweiten Rolle anzunähern, wobei eine erste Grenzkraft auf den Handgriff ein Abdichten des nahe liegenden Abschnitts der Fuhrungsdrahtbaugruppe verursacht, und eine zweite Grenzkraft auf den Handgriff ein Abtrennen des nahe liegenden Abschnitts der Fuhrungsdrahtbaugruppe verursacht.

19. System gemäß Anspruch 19, wobei der Handgriff fur eine automatische Rückkehr in eine geoffnete Anfangsposition bei der Beendigung einer Kraft, federvorgespannt ist.

## Revendications

1. Système d'occlusion à fil de guidage (20) destiné à être utilisé dans des procédures vasculaires, comprenant:
(a) un assemblage tubulaire à fil de guidage comprenant un ballon occlusif (32) à proximité d'une extrémité distale (26) et une section scellable étendue (24) à proximité d'une extrémité proximale ;
(b) un système de gonflage/évacuation à gaz (80) pouvant être connecté de manière amovible à l'extrémité proximale de l'assemblage tubulaire à fil de guidage, comprenant :
(1) des moyens pour l'évacuation de l'assemblage tubulaire à fil de guidage ; et
(2) des moyens pour l'introduction d'un gaz dans l'assemblage tubulaire à fil de guidage afin de gonfler le ballon occlusif une pluralité de fois ; et
(c) un système de scellement (68) pouvant être connecté de manière amovible à l'extrémité proximale (36) de l'assemblage tubulaire à fil de guidage, comprenant :
(1) des moyens pour le scellement sélectif de l'assemblage tubulaire à fil de guidage en formant des joints successifs étanches à l'air à des endroits séparés le long de la section scellable étendue afin de retenir le gaz dans le ballon occlusif une pluralité de fois, le diamètre de la section scellable étendue n'étant pas supérieur, lorsqu'elle est scellée, au diamètre de tout autre portion de l'assemblage tubulaire à fil de guidage, de façon à ce que l'assemblage tubulaire à fil de guidage puisse être utilisé en tant que fil de guidage pour introduire un cathéter par l'intermédiaire du fil de guidage tandis que le gaz est retenu dans le ballon occlusif ;
**caractérisé en ce que** la section scellable étendue est constituée d'une section sertissable étendue (28) et les moyens pour le scellement sélectif comprennent un mécanisme de sertissage (66) qui déforme et sertit la section sertissable étendue de façon à former un joint étanche à l'air.

2. Système de la revendication 1, dans lequel l'assemblage tubulaire à fil de guidage présente une longueur effective d'au moins 40 cm et un diamètre extérieur inférieur à 0,02 cm (0,06 pouce), dans lequel la section scellable étendue présente une longueur effective d'au moins 1 cm et un diamètre extérieur inférieur à 0,13 cm (0,05 pouce) et dans lequel le ballon occlusif peut être dégonflé en moins de deux minutes

3. Système de la revendication 1, dans lequel l'assemblage tubulaire à fil de guidage présente une longueur effective d'au moins 100 cm et un diamètre extérieur inférieur à 0,05 cm (0,018 pouce), dans lequel la section scellable étendue présente une longueur effective d'au moins 5 cm et un diamètre extérieur inférieur à 0,03 cm (0,012 pouce) et dans lequel le ballon occlusif peut être dégonflé en moins d'une minute.

4. Système de l'une des revendications précédentes, dans lequel la section sertissable étendue est dimensionnée et le mécanisme de sertissage est conçu de façon à ce qu'un diamètre extérieur effectif de la section sertissable étendue au niveau d'un joint ne soit pas supérieur au diamètre extérieur d'une portion du corps principal de l'assemblage tubulaire à fil de guidage lorsque la section sertissable étendue est sertie au niveau du joint.

5. Système de l'une des revendications précédentes, dans lequel le ballon occlusif peut être gonflé et dégonflé de manière répétitive au cours d'une procédure vasculaire, et entre ces gonflements et dégonflements répétés, l'extrémité proximale de l'assemblage tubulaire à fil de guidage est exempte de connexions mécaniques et d'obstructions, ce qui permet à l'assemblage tubulaire à fil de guidage de fonctionner comme un fil de guidage conventionnel pour des échanges de cathéters.

6. Système de l'une des revendications précédentes, comprenant en outre des moyens pour l'ouverture sélective de la section scellable étendue distale par rapport à chaque joint étanche à l'air permanent successif, afin de dégonfler le ballon occlusif.

7. Système de l'une des revendications précédentes, comprenant :
(1) une unité portative comprenant un mécanisme de sertissage ayant une première ouverture et un mécanisme de scellement ayant une seconde ouverture, un passage s'étendant de la première ouverture à la seconde ouverture pour recevoir l'extrémité proximale de l'assemblage tubulaire à fil de guidage ,
(2) un système à seringue d'évacuation ;
(3) un système à seringue de gonflage contenant un volume de gaz suffisant pour gonfler le ballon occlusif une pluralité de fois , et
(4) des conduits reliant, de manière opérationnelle, le système à seringue d'évacuation et le système à seringue de gonflage à la seconde ouverture de l'unité portative, les conduits comprenant un dispositif à vanne qui relie de manière sélective soit le système à seringue d'évacuation soit le système à seringue de gonflage à la seconde ouverture.

8. Système de la revendication 7, dans lequel le système à seringue de gonflage comprend une pluralité de seringues individuelles, chaque seringue individuelle contenant un volume de gaz suffisant pour gonfler le ballon occlusif une fois.

9. Système de la revendication 7 ou 8, dans lequel le conduit relié au système à seringue d'évacuation comprend un clapet anti-retour permettant seulement l'évacuation et le conduit relié au système à seringue de gonflage comprend un clapet anti-retour permettant seulement le gonflage.

10. Système de l'une des revendications précédentes, dans lequel l'élément tubulaire à fil de guidage comprend une portion principale de corps constituée d'un hypotube en acier inoxydable.

11. Système de la revendication 10, dans lequel la section sertissable étendue est fixée de manière colinéaire à l'extrémité proximale de l'hypotube en acier inoxydable et est constituée d'un hypotube en acier inoxydable ayant un diamètre extérieur inférieur au diamètre extérieur de l'hypotube en acier inoxydable de la portion principale du corps.

12. Système de la revendication 10 ou 11 dans lequel l'élément tubulaire à fil de guidage comprend en outre une portion distale constituée d'un alliage Ni-Ti.

13. Système de l'une des revendications précédentes, dans lequel une section sertissable étendue est affaiblie par des stries au niveau d'une pluralité d'endroits le long de la section sertissable étendue afin de pouvoir découper la section sertissable étendue au niveau de ces endroits

14. Système de l'une des revendications précédentes, dans lequel l'élément tubulaire à fil de guidage et la section sertissable étendue sont constitués d'un tube continu en acier inoxydable entouré par un fil excepté le long de la section sertissable étendue.

15. Système de l'une des revendications précédentes, dans lequel l'élément tubulaire à fil de guidage comprend un hypotube tressé en polyimide ayant une tige centrale conique à l'intérieur.

16. Système de la revendication 15, dans lequel au moins une portion proximale de la tige centrale conique est tubulaire, elle possède un lumen défini à l'intérieur et s'étend de manière proximale par rapport à une extrémité proximale de l'hyoptube tressé en polyimide afin de définir la section sertissable étendue.

17. Système de l'une des revendications précédentes, comprenant en outre une paire de bobines positionnées le long d'une portion distale de l'élément tubulaire à fil de guidage, espacées longitudinalement et adjacentes par rapport à au moins un trou dans l'élément tubulaire à fil de guidage qui permet un accès du fluide entre le lumen et le ballon occlusif, au moins une portion du ballon occlusif étant reliée à au moins une portion de chacune des bobines.

18. Système de l'une des revendications précédentes, dans lequel le mécanisme de sertissage comprend :
un premier rouleau et un second rouleau, espacé de manière proximale par rapport au premier rouleau, pour la traversée de la portion proximale de l'assemblage à fil de guidage, le premier rouleau étant relié à un manche avec un dispositif à came pivotante de façon à ce qu'une force exercée sur le manche provoque l'approche proportionnelle du premier rouleau par rapport au second rouleau, un premier seuil de force sur le manche provoquant le scellement de la portion proximale de l'assemblage à fil de guidage et un second seuil de force sur le manche provoquant le découpage de la portion proximale de l'assemblage à fil de guidage.

19. Système de la revendication 18 dans lequel le manche est précontraint par un ressort pour un retour automatique vers une position de départ ouverte lorsqu'aucune force n'est exercée
